(19) Europäisches Patentamt
European Patent Office
Office européen des brevets

(11) EP 2 363 110 B2

(12) # NOUVEAU FASCICULE DE BREVET EUROPEEN

Après la procédure d'opposition

(45) Date de publication et mention de la décision concernant l'opposition:
**02.08.2017 Bulletin 2017/31**

(45) Mention de la délivrance du brevet:
**23.10.2013 Bulletin 2013/43**

(21) Numéro de dépôt: **10175970.2**

(22) Date de dépôt: **09.09.2010**

(51) Int Cl.:
***A61K 8/92*** *(2006.01)* ***A61Q 5/00*** *(2006.01)*
***A61Q 5/10*** *(2006.01)*

(54) **Utilisation d'une huile siccative pour protéger la couleur vis-à-vis du lavage de fibres keratiniques teintes artificiellement; procédés de coloration**

Verwendung von trocknenden Ölen zum Schutz der Farbe künstlich gefärbt Keratinfasern gegen Waschen; Methode zur Färbung von Haaren

Use of drying oils for protecting the color of artificially colored keratin fibers against washing; method for coloring hair

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **15.09.2009 FR 0956336**

(43) Date de publication de la demande:
**07.09.2011 Bulletin 2011/36**

(73) Titulaire: **L'Oréal**
**75008 Paris (FR)**

(72) Inventeurs:
- **Neplaz Stéphanie**
  **75011, Paris (FR)**
- **Fack, Géraldine**
  **92300, Levallois (FR)**
- **Lazzeri-Vigouroux, Pascale**
  **13540, Puyricard (FR)**
- **Mellul, Myriam**
  **75014, Paris (FR)**

(74) Mandataire: **Dodin, Catherine**
**L'Oréal**
**Service DIPI**
**9 Rue Pierre Dreyfus**
**92110 Clichy (FR)**

(56) Documents cités:
**EP-A2- 1 240 831**
**WO-A1-95/22312**
**FR-A- 1 299 757**
**GB-A- 769 082**
**GB-A- 2 069 335**
**US-A- 3 211 618**
**US-A1- 2009 071 494**

EP 2 363 110 B2

## Description

L'invention a pour objet l'utilisation d'une ou plusieurs huiles siccatives choisies parmi l'huile de lin, l'huile de bois de Chine (ou Canton) appelée aussi huile de tung, l'huile d'oïticica, l'huile de vernonia, l'huile d'oeillette, l'huile de grenade, l'huile de calendula, l'huile de Perilla et leurs mélanges comme agent permettant de protéger la couleur vis-à-vis du lavage des fibres kératiniques teintes par coloration directe ou par coloration d'oxydation, notamment des fibres kératiniques humaines et plus particulièrement des cheveux.

Il est connu de teindre les fibres kératiniques notamment humaines et en particulier les cheveux avec des compositions tinctoriales contenant des précurseurs de colorant d'oxydation, appelés généralement bases d'oxydation. Ces bases d'oxydation sont des composés incolores ou faiblement colorés qui, associés à des produits oxydants, donnent naissance par un processus de condensation oxydative à des composés colorés. On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Il est aussi connu de teindre les fibres kératiniques par une coloration directe. Le procédé classiquement utilisé en coloration directe consiste à appliquer sur les fibres kératiniques des colorants directs qui sont des molécules colorées et colorantes ayant une affinité pour les fibres, à laisser poser, puis à rincer les fibres. Cette coloration directe peut être éventuellement effectuées en présence d'un agent oxydant, on parle alors de coloration directe éclaircissante.

Les colorations qui en résultent sont des colorations particulièrement chromatiques qui sont cependant temporaires ou semi-permanentes car la nature des interactions qui lient les colorants directs à la fibre kératinique et leur désorption de la surface et/ou du coeur de la fibre sont responsables de leur faible puissance tinctoriale et de leur mauvaise tenue aux lavages.

La couleur artificielle des cheveux apportée par un traitement de coloration directe ou d'oxydation s'estompe progressivement du fait des lavages répétés et conduit dans le temps à un affadissement de la coloration des cheveux. L'utilisation des produits soins rincés et non rincés commercialisés n'améliore pas suffisamment la tenue de la couleur artificielle des cheveux.

Il est donc nécessaire de mettre au point des moyens permettant de protéger la couleur artificielle de l'effet des lavages répétés, c'est-à-dire d'améliorer la tenue de la couleur artificielle des cheveux.

On connaît la demande EP1312346 qui décrit l'utilisation de silicones aminées pour protéger la couleur des cheveux et la demande EP 1 240 831 qui décrit l'utilisation des extraits végétaux pour protéger la couleur des cheveux.

La Demanderesse a maintenant découvert de manière surprenante que l'utilisation d'une ou plusieurs huiles siccatives choisies parmi l'huile de lin, l'huile de bois de Chine (ou Canton) appelée aussi huile de tung, l'huile d'oïticica, l'huile de vernonia, l'huile d'oeillette, l'huile de grenade, l'huile de calendula, l'huile de Perilla et leurs mélanges permettait de protéger vis-à-vis du lavage la couleur artificielle des fibres kératiniques teintes par coloration directe ou par coloration d'oxydation.

Cette découverte est à la base de la présente invention.

On entend au sens de l'invention par « agent oxydant », tout composé ayant des propriétés oxydantes et étant différent de l'oxygène de l'air.

On entend par « fibres kératiniques humaines » les cheveux, les poils notamment de barbe ou moustache, les cils, les sourcils.

On entend par « fibres kératiniques teintes artificiellement » des fibres kératiniques teintes par un procédé de coloration directe ou par un procédé de coloration d'oxydation.

On entend par « lavage », une ou plusieurs applications sur les fibres kératiniques d'une composition aqueuse éliminée, le plus souvent détergente telle qu'un shampooing. Cette expression inclut également les baignades en particulier en mer ou en piscine.

L'invention a donc pour objet l'utilisation cosmétique d'une ou plusieurs huiles siccatives choisies parmi l'huile de lin, l'huile de bois de Chine (ou Canton) appelée aussi huile de tung, l'huile d'oïticica, l'huile de vernonia, l'huile d'oeillette, l'huile de grenade, l'huile de calendula, l'huile de Perilla et leurs mélanges comme agent permettant de protéger vis-à-vis du lavage la couleur des fibres kératiniques teintes artificiellement, notamment des fibres kératiniques humaines et plus particulièrement des cheveux.

De manière préférentielle, les fibres kératiniques sont teintes par coloration d'oxydation, notamment en présence d'un ou plusieurs agents oxydants.

L'invention a également pour objet un procédé pour protéger la couleur vis-à-vis du lavage des fibres kératiniques teintes artificiellement, caractérisé par le fait qu'il consiste à appliquer sur lesdites fibres, une ou plusieurs huiles siccatives choisies parmi l'huile de lin, l'huile de bois de Chine (ou Canton) appelée aussi huile de tung, l'huile d'oïticica, l'huile de vernonia, l'huile d'oeillette, l'huile de grenade, l'huile de calendula, l'huile de Perilla et leurs mélanges ou une composition comprenant dans un milieu cosmétiquement acceptable une ou plusieurs huiles siccatives choisies parmi l'huile de lin, l'huile de bois de Chine (ou Canton) appelée aussi huile de tung, l'huile d'oïticica, l'huile de vernonia, l'huile

d'oeillette, l'huile de grenade, l'huile de calendula, l'huile de Perilla et leurs mélanges. De manière préférentielle, les fibres kératiniques sont teintes par coloration d'oxydation en présence d'un ou plusieurs agents oxydants.

La ou les huiles siccatives peuvent être introduites dans la composition tinctoriale appliquée sur les fibres kératiniques.

Elles peuvent être introduites de préférence dans une composition appliquée avant ou après teinture des fibres kératiniques. Plus préférentiellement, la ou les huiles siccatives sont introduites dans une composition appliquée après teinture des fibres kératiniques, c'est-à-dire sur des cheveux préalablement teints artificiellement.

De plus, la protection apportée par le traitement selon l'invention est durable c'est-à-dire ne nécessitant pas de réapplications fréquentes du produit.

Un autre objet de l'invention concerne un procédé de coloration consistant à appliquer sur les fibres kératiniques humaines et plus particulièrement les cheveux, une composition (A) colorante directe ou une composition (A) colorante d'oxydation pendant un temps suffisant pour développer la couleur, et de faire suivre ou précéder cette application par l'application d'une composition (B) contenant dans un milieu cosmétiquement acceptable une ou plusieurs huiles siccatives choisies parmi l'huile de lin, l'huile de bois de Chine (ou Canton) appelée aussi huile de tung, l'huile d'oïticica, l'huile de vernonia, l'huile d'oeillette, l'huile de grenade, l'huile de calendula, l'huile de Perilla et leurs mélanges. De manière préférentielle, la composition (A) est une composition colorante d'oxydation en présence d'un ou plusieurs agents oxydants.

Un autre objet de l'invention concerne un procédé de coloration consistant à appliquer sur les fibres kératiniques en particulier les fibres kératiniques humaines et plus particulièrement les cheveux, une composition (A) colorante directe pendant un temps suffisant pour développer la couleur, ladite composition (A) comprenant dans un milieu cosmétiquement acceptable une ou plusieurs huiles siccatives choisies parmi l'huile de lin, l'huile de bois de Chine (ou Canton) appelée aussi huile de tung, l'huile d'oïticica, l'huile de vernonia, l'huile d'oeillette, l'huile de grenade, l'huile de calendula, l'huile de Perilla et leurs mélanges.

Les différents objets de l'invention vont maintenant être détaillés. L'ensemble des significations et définitions des composés utilisés dans la présente invention données ci-dessous sont valables pour l'ensemble des objets de l'invention.

Le milieu cosmétiquement acceptable des compositions protectrices de la couleur selon l'invention peut par exemple être constitué par de l'eau, par un ou plusieurs solvants organiques, par des huiles différentes des huiles siccatives ou par un mélange d'eau et d'au moins un solvant organique cosmétiquement acceptable. A titre de solvants organiques, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol et leurs mélanges. A titre d'huiles différentes des huiles siccatives, on peut citer les huiles végétales non siccatives, les huiles minérales et l'huile de vaseline.

## Huiles siccatives

Par « huile siccative », on entend désigner une huile qui, lorsque étalée en couche mince puis exposée à l'air, se réticule et se transforme en film rigide voire solide.

En particulier, on entend désigner dans le cadre de la présente invention par « huile siccative », les huiles, et de préférence les triglycérides, comportant des doubles liaisons, de préférence comportant au moins deux doubles liaisons, et de préférence comportant au moins trois doubles liaisons. Les doubles laisons peuvent être conjuguées ou non conjuguées.

Les huiles siccatives selon l'invention ont un indice d'iode supérieur ou égal à 90, de préférence allant de 100 à 200.

Les huiles siccatives conformes à l'invention peuvent être d'origine naturelle et sont choisies parmi l'huile de lin, l'huile de bois de Chine (ou Canton) appelée aussi huile de tung, l'huile d'oïticica, l'huile de vernonia, l'huile d'oeillette, l'huile de grenade, l'huile de calendula, l'huile de Perilla et leurs mélanges.

L'huile siccative convenant à la mise en oeuvre de la présente invention peut être modifiée par action physique ou chimique.

En particulier, elle peut être raffinée et/ou partiellement polymérisée. A ce titre, on peut citer les huiles soufflées et les standolies, les huiles maléinisées, époxydées ou cuites.

Selon un mode de réalisation particulier de l'invention, l'huile siccative est une huile de lin raffinée.

Une huile peut être raffinée en particulier en trois étapes successives.

L'huile de lin raffinée selon l'invention peut ainsi résulter d'une étape de dégommage, pour obtenir en particulier une huile démucilaginée, suivie d'une étape de décoloration, en particulier pour la blanchir, puis d'une étape de neutralisation.

Selon un mode particulier de réalisation de l'invention, l'huile siccative de l'invention est une huile de lin modifiée selon au moins l'une des trois étapes mentionnées précédemment, autrement dit qui a subi soit une étape de dégommage,

soit une étape de décoloration, soit une étape de neutralisation, soit une succession de plusieurs de ces étapes.

Selon un autre mode de réalisation particulier de l'invention, l'huile siccative est une huile siccative en particulier de lin soufflée ou polymérisée à chaud.

Le soufflage d'une huile se caractérise notamment par une polymérisation partielle de ladite huile avec l'oxygène de l'air. L'huile soufflée peut être en particulier obtenue en soufflant de l'air au travers de l'huile chauffée.

La polymérisation est obtenue par chauffage sous atmosphère inerte en particulier à des températures allant de 250°C à 300°C et notamment proche de 280°C pour l'huile de lin. Les huiles ainsi modifiées sont appelées des standolies.

Les huiles siccatives sont notamment choisies parmi l'huile de lin native ou raffinée et les standolies de lin (standolies d'huile de lin).

La ou les huiles siccatives conformes à l'invention peuvent être présents dans les compositions protectrices de la couleur dans des concentrations allant de préférence de 0,05 à 100% en poids et plus préférentiellement de 0,1 à 40% en poids et encore plus préférentiellement de 0,1 à 30% en poids, mieux de 0,5 à 10 % en poids par rapport au poids total de la composition.

Les solvants organiques précités sont, de préférence, présents dans des proportions de préférence allant de 1 à 95 % en poids environ par rapport au poids total de la composition, et encore plus préférentiellement de 1 à 90 et mieux de 3 à 30 % en poids environ.

La composition selon l'invention contenant l'agent ou les agents protecteurs de la couleur des fibres kératiniques peut également renfermer divers adjuvants utilisés classiquement dans les compositions de traitement capillaire, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents de pénétration, des agents séquestrants, des parfums, des tampons, des agents dispersants, des agents conditionneurs tels que par exemple des silicones volatiles ou non volatiles, modifiées ou non modifiées, des agents filmogènes, des céramides, des agents conservateurs, des agents opacifiants.

Les compositions selon l'invention peuvent en particulier contenir un ou plusieurs agents conditionneurs dont certains peuvent aussi être des solvants organiques.

Dans le cadre de la présente invention, on entend par « agent conditionneur » tout agent ayant pour fonction l'amélioration des propriétés cosmétiques des cheveux, en particulier la douceur, le démêlage, le toucher, le lissage, l'électricité statique.

Les agents conditionneurs peuvent se présenter sous forme liquide, semi-solide ou solide tels que par exemple des huiles, des cires ou des gommes.

Selon l'invention, les agents conditionneurs peuvent être choisis parmi les huiles de synthèses telles que les poly-oléfines, les huiles végétales non siccatives, les huiles fluorées ou perfluorées, les cires naturelles ou synthétiques, les silicones, les polymères cationiques, les composés de type céramide, les tensioactifs cationiques, les amines grasses, les acides gras ou les esters d'acides gras autres que les huiles végétales ainsi que les mélanges de ces différents composés.

Les huiles de synthèse sont notamment les polyoléfines en particulier les poly-$\alpha$-oléfines et plus particulièrement :

- de type polybutène, hydrogéné ou non, et de préférence polyisobutène, hydrogéné ou non.

On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.

A titre d'exemples de poly-$\alpha$-oléfines utilisables dans le cadre de la présente invention, on peut plus particulièrement mentionner les polyisobutènes vendus sous le nom de PERMETHYL 99 A, 101 A, 102 A, 104 A (n=16) et 106 A (n=38) par la Société PRESPERSE Inc, ou bien encore les produits vendus sous le nom de ARLAMOL HD (n=3) par la Société ICI (n désignant le degré de polymérisation),

- de type polydécène, hydrogéné ou non.

De tels produits sont vendus par exemple sous les dénominations ETHYLFLO par la société ETHYL CORP., et d'ARLAMOL PAO par la société ICI.

Les huiles animales ou végétales sont choisies préférentiellement dans le groupe formé par les huiles de tournesol, de maïs, de soja, d'avocat, de jojoba, de courge, de pépins de raisin, de sésame, de noisette, les huiles de poisson, le tricaprocaprylate de glycérol, ou les huiles de formule (I) $R_9COOR_{10}$ dans laquelle $R_9$ représente un radical alkyle comportant de 7 à 29 atomes de carbone et $R_{10}$ représente une chaîne hydrocarbonée linéaire ou ramifiée contenant de 3 à 30 atomes de carbone en particulier alkyle ou alkényle, par exemple, l'huile de Purcellin.

Les cires sont des substances naturelles (animales ou végétales) ou synthétiques solides à température ambiante (20°-25°C). Elles sont insolubles dans l'eau, solubles dans les huiles et sont capables de former un film hydrofuge.
Sur la définition des cires, on peut citer par exemple P.D. Dorgan, Drug and Cosmetic Industry, Decembre 1983, pp. 30-33.
La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la Société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.
Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519 863.
De manière encore plus générale, au sens de la présente invention, l'expression "polymère cationique" désigne tout polymère contenant des groupements cationiques et/ou des groupements ionisables en groupements cationiques.
Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.
Les polymères cationiques utilisés ont généralement une masse moléculaire moyenne en nombre comprise entre 500 et $5.10^6$ environ, et de préférence comprise entre $10^3$ et $3.10^6$ environ.
Parmi les polymères cationiques, on peut citer plus particulièrement les polymères du type polyamine, polyaminoamide et polyammonium quaternaire, les polymères cationiques dérivés de polysaccharides. Ce sont des produits connus.
Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :

(1) les homopolymères ou copolymères dérivés d'esters ou d'amides acryliques ou méthacryliques et comportant au moins un des motifs de formules suivantes:

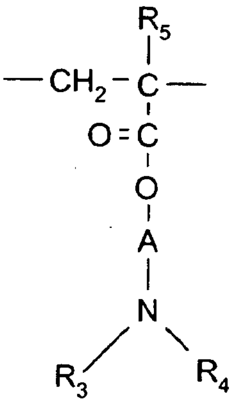 (I), 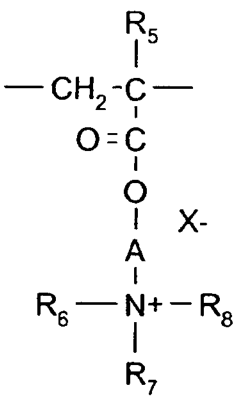 (II)

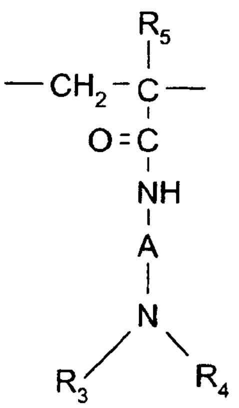

(III)

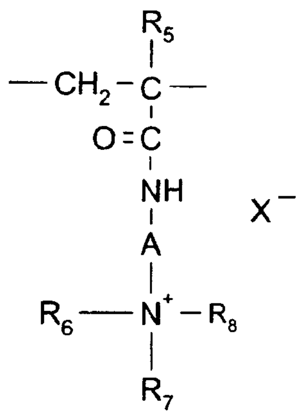

(IV)

dans lesquelles:

$R_3$ et $R_4$, identiques ou différents, représentent hydrogène ou un groupe alkyle ayant de 1 à 6 atomes de carbone et de préférence méthyle ou éthyle;
$R_5$, identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_6$, $R_7$, $R_8$, identiques ou différents, représentent un groupe alkyle ayant de 1 à 18 atomes de carbone ou un radical benzyle et de préférence un groupe alkyle ayant de 1 à 6 atomes de carbone;
X désigne un anion dérivé d'un acide minéral ou organique tel que un anion méthosulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs ($C_1$-$C_4$), des acides acryliques ou méthacryliques ou leurs esters, des vinyllactames tels que la vinylpyrrolidone ou le vinylcaprolactame, des esters vinyliques.
Ainsi, parmi ces copolymères de la famille (1), on peut citer :

- les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
- les copolymères d'acrylamide et de chlorure de méthacryloyloxyéthyltriméthyl-ammonium décrit par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthyl-ammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP.
- et les copolymères vinylpyrrolidone / méthacrylamide de diméthylaminopropyle quaternisé tel que le produit vendu sous la dénomination "GAFQUAT HS 100" par la société ISP, et
- les polymères réticulés de sels de méthacryloyloxyalkyl ($C_1$-$C_4$) trialkyl ($C_1$-$C_4$) ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant

50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium par exemple en dispersion dans de l'huile minérale ou dans un ester liquide. Ces dispersions sont commercialisées sous les noms de « SALCARE® SC 95 » et « SALCARE® SC 96 » par la Société CIBA.

(2) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;

(3) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;

(4) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(5) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

(6) les cyclopolymères d'alkyl diallyl amine ou de dialkyl diallyl ammonium tels que les homopolymères ou copolymères comportant comme constituant principal de la chaîne des motifs répondant aux formules (V) ou (VI) :

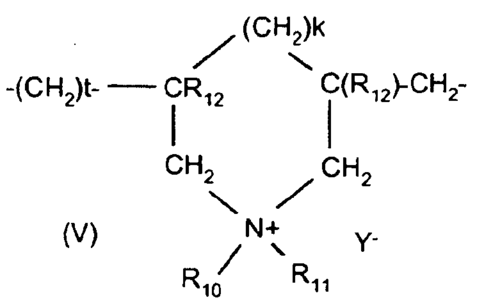
(V)

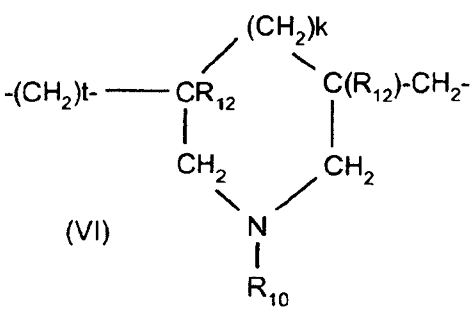
(VI)

formules dans lesquelles k et t sont égaux à 0 ou 1, la somme k + t étant égale à 1 ; $R_{12}$ désigne un atome d'hydrogène ou un radical méthyle ; $R_{10}$ et $R_{11}$, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur ($C_1$-$C_4$) ou $R_{10}$ et $R_{11}$ peuvent désigner conjointement avec l'atome

d'azote auquel ils sont rattachés, des groupement hétérocycliques, tels que pipéridinyle ou morpholinyle ; $Y^-$ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
$R_{10}$ et $R_{11}$, indépendamment l'un de l'autre, désignent de préférence un groupement alkyle ayant de 1 à 4 atomes de carbone.
Parmi les polymères définis ci-dessus, on peut citer plus particulièrement l'homopolymère de chlorure de diméthyldiallylammonium vendu sous la dénomination "Merquat 100" par la société NALCO (et ses homologues de faibles masses moléculaires moyenne en poids) et les copolymères de chlorure de diallyldiméthylammonium et d'acrylamide commercialisés sous la dénomination "MERQUAT 550".

(7) le polymère de diammonium quaternaire contenant des motifs récurrents répondant à la formule :

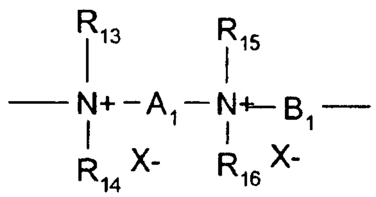

(VII)

formule (VII) dans laquelle :

$R_{13}$, $R_{14}$, $R_{15}$ et $R_{16}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{13}$, $R_{14}$, $R_{15}$ et $R_{16}$, ensemble ou séparément, constituent avec les atomes d'azote auxquels ils sont rattachés des hétérocycles contenant éventuellement un second hétéroatome autre que l'azote ou bien $R_{13}$, $R_{14}$, $R_{15}$ et $R_{16}$ représentent un radical alkyle en $C_1$-$C_6$ linéaire ou ramifié substitué par un groupement nitrile, ester, acyle, amide ou -CO-O-$R_{17}$-D ou -CO-NH-$R_{17}$-D où $R_{17}$ est un alkylène et D un groupement ammonium quaternaire ;
$A_1$ et $B_1$ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et X- désigne un anion dérivé d'un acide minéral ou organique;
$A_1$, $R_{13}$ et $R_{15}$ peuvent former avec les deux atomes d'azote auxquels ils sont rattachés un cycle pipérazinique ; en outre si $A_1$ désigne un radical alkylène ou hydroxyalkylène linéaire ou ramifié, saturé ou insaturé, $B_1$ peut également désigner un groupement $(CH_2)_n$-CO-D-OC-$(CH_2)$ p- n et p sont des nombres entiers variant de 2 à 20 environ
dans lequel D désigne :

a) un reste de glycol de formule : -O-Z-O-, où Z désigne un radical hydrocarboné linéaire ou ramifié ou un groupement répondant à l'une des formules suivantes :

$$-(CH_2-CH_2-O)_x-CH_2-CH_2-$$

$$-[CH_2-CH(CH_3)-O]y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;

b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2- ;$$

d) un groupement uréylène de formule : -NH-CO-NH- ;

De préférence, X- est un anion tel que le chlorure ou le bromure.

Ces polymères ont une masse moléculaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
On peut utiliser plus particulièrement les polymères qui sont constitués de motifs récurrents répondant à la formule :

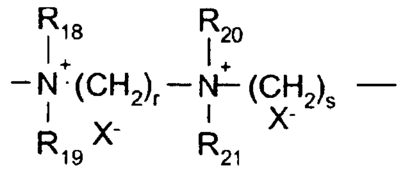
(VIII)

dans laquelle $R_{18}$, $R_{19}$, $R_{20}$ et $R_{21}$, identiques ou différents, désignent un radical alkyle ou hydroxyalkyle ayant de 1 à 4 atomes de carbone environ, r et s sont des nombres entiers variant de 2 à 20 environ et, X- est un anion dérivé d'un acide minéral ou organique.
Un composé de formule (VIII) particulièrement préféré est celui pour lequel $R_{18}$, $R_{19}$, $R_{20}$ et R21, représentent un radical méthyle et r = 3, s = 6 et X = Cl, dénommé Hexadimethrine chloride selon la nomenclature INCI (CTFA).

(8) les polymères de polyammonium quaternaires constitués de motifs de formule (IX):

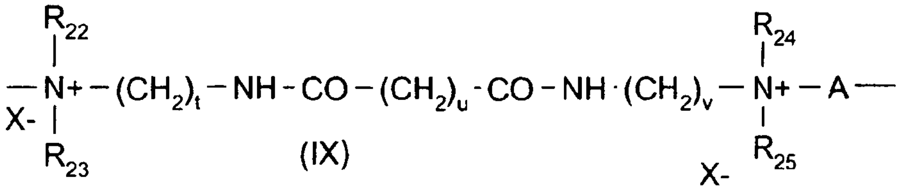
(IX)

formule dans laquelle :

$R_{22}$, $R_{23}$, $R_{24}$ et $R_{25}$, identiques ou différents, représentent un atome d'hydrogène ou un radical méthyle, éthyle, propyle, β-hydroxyéthyle, β-hydroxypropyle ou $-CH_2CH_2(OCH_2CH_2)_pOH$,
où p est égal à 0 ou à un nombre entier compris entre 1 et 6, sous réserve que $R_{22}$, $R_{23}$, $R_{24}$ et $R_{25}$ ne représentent pas simultanément un atome d'hydrogène,
t et u, identiques ou différents, sont des nombres entiers compris entre 1 et 6,
v est égal à 0 ou à un nombre entier compris entre 1 et 34,
$X^-$ désigne un anion tel qu'un halogènure,
A désigne un radical d'un dihalogénure ou représente de préférence $-CH_2-CH_2-O-CH_2-CH_2-$.
De tels composés sont notamment décrits dans la demande de brevet EP-A-1 22 324.
On peut par exemple citer parmi ceux-ci, les produits "Mirapol® A 15", "Mirapol® AD1", "Mirapol® AZ1" et "Mirapol® 175" vendus par la société Miranol.

(9) Les polymères quaternaires de vinylpyrrolidone et de vinylimidazole tels que par exemple les produits commercialisés sous les dénominations Luviquat® FC 905, FC 550 et FC 370 par la société B.A.S.F.

(10) Les polysaccharides cationiques notamment les celluloses et les gommes de galactomannanes cationiques.

Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
Les dérivés d'éthers de cellulose comportant des groupements ammonium quaternaires décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.
Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses,

comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.

Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Les protéines ou hydrolysats de protéines cationiques sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :

- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate" ;
- les hydrolysats de collagène portant des groupements chlorure de triméthylammonium et de triméthylstéarylammonium, vendus sous la dénomination de "Quat-Pro S" par la Société MAYBROOOK et dénommés dans le dictionnaire CTFA "Steartrimonium Hydrolyzed Collagen" ;
- les hydrolysats de protéines animales portant des groupements triméthylbenzylammonium tels que les produits vendus sous la dénomination "Crotein BTA" par la Société CRODA et dénommés dans le dictionnaire CTFA "Benzyltrimonium hydrolyzed animal protein" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :

- le "Croquat L" dont les groupements ammonium quaternaires comportent un groupement alkyle en $C_{12}$ ;
- le "Croquat M" dont les groupements ammonium quaternairees comportent des groupements alkyle en $C_{10}$-$C_{18}$;
- le "Croquat S" dont les groupements ammonium quaternaires comportent un groupement alkyle en $C_{18}$ ;
- le "Crotein Q" dont les groupements ammonium quaternaires comportent au moins un groupe alkyle ayant de 1 à 18 atomes de carbone.

Ces différents produits sont vendus par la Société Croda.

D'autre protéines ou hydrolysats quaternisés sont par exemple ceux répondant à la formule (X) :

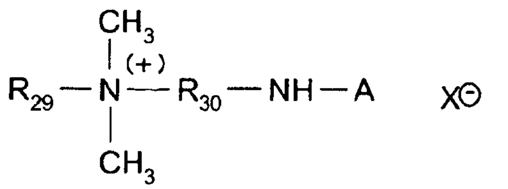
(X)

dans laquelle X- est un anion d'un acide organique ou minéral, A désigne un reste de protéine dérivé d'hydrolysats de protéine de collagène, $R_{29}$ désigne un groupement lipophile comportant jusqu'à 30 atomes de carbone, $R_{30}$ représente un groupement alkylène ayant 1 à 6 atomes de carbone. On peut citer par exemple les produits vendus par la Société Inolex, sous la dénomination "Lexein QX 3000", appelé dans le dictionnaire CTFA "Cocotrimonium Collagent Hydrolysate".

On peut encore citer les protéines végétales quaternisées telles que les protéines de blé, de maïs ou de soja : comme protéines de blé quaternisées, on peut citer celles commercialisées par la Société Croda sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Parmi tous les polymères cationiques susceptibles d'être utilisés dans le cadre de la présente invention, on préfère mettre en oeuvre les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole, les polysaccharides

cationiques et leurs mélanges.

Les silicones utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :

(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.

On peut également citer les cyclocopolymères du type diméthylsiloxanes/méthylakylsiloxane, tel que la "SILICONE VOLATILE FZ 3109" commercialisée par la société UNION CARBIDE, de structure chimique :

avec D :

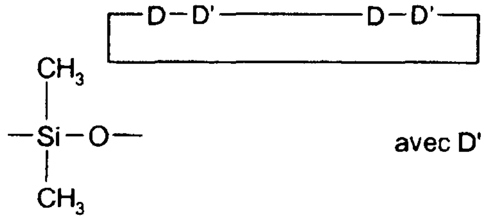

avec D' :

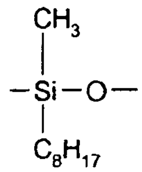

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy)bis-néopentane ;

(ii) les silicones volatiles linéaires ayant 2 à 9 atomes de silicium et possédant une viscosité inférieure ou égale à $5.10^{-6} m^2/s$ à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de $5.10^{-6}$ à 2,5 $m^2/s$ à 25°C et de préférence $1.10^{-5}$ à 1 $m^2/s$. La viscosité des silicones est par exemple mesurée à 25°C selon la norme ASTM 445 Appendice C.

Parmi ces polyalkylsiloxanes, on peut citer à titre non limitatif les produits commerciaux suivants :

- les huiles SILBIONE des séries 47 et 70 047 ou les huiles MIRASIL commercialisées par RHODIA CHIMIE telles que par exemple l'huile 70 047 V 500 000 ;
- les huiles de la série MIRASIL commercialisées par la société RHODIA CHIMIE
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE.

Dans cette classe de polyalkylsiloxanes, on peut également citer les produits commercialisés sous les dénominations "ABIL WAX 9800 et 9801" par la société GOLDSCHMIDT qui sont des polyalkyl ($C_1$-$C_{20}$) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de $1.10^{-5}$ à $5.10^{-2} m^2/s$ à 25°C.

Parmi ces polyalkylarylsiloxanes on peut citer à titre d'exemple les produits commercialisés sous les dénominations suivantes :

- les huiles SILBIONE de la série 70 641 de RHODIA CHIMIE;
- les huiles des séries RHODORSIL 70 633 et 763 de RHODIA CHIMIE;
- l'huile DOW CORNING 556 COSMETIC GRAD FLUID de DOW CORNING;
- les silicones de la série PK de BAYER comme le produit PK20 ;
- les silicones des séries PN, PH de BAYER comme les produits PN1000 et PH1000;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.
On peut plus particulièrement citer les produits suivants :

- les gommes polydiméthylsiloxane
- les gommes polydiméthylsiloxanes/méthylvinylsiloxane,
- les gommes polydiméthylsiloxane/diphénylsiloxane,
- les gommes polydiméthylsiloxane/phénylméthylsiloxane,
- les gommes polydiméthylsiloxane/diphénylsiloxane/méthylvinylsiloxane.

Des produits plus particulièrement utilisables conformément à l'invention sont des mélanges tels que :

- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6} m^2/s$. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
$R_2SiO_{2/2}$, $R_3SiO_{1/2}$, $RSiO_{3/2}$ et $SiO_{4/2}$ dans lesquelles R représente un groupement hydrocarboné possédant 1 à 16 atomes de carbone ou un groupement phényle.
Parmi ces produits, ceux particulièrement préférés sont ceux dans lesquels R désigne un radical alkyle inférieur en $C_1$-$C_4$, plus particulièrement méthyle, ou un radical phényle.
On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.
On peut également citer les résines du type triméthylsiloxysilicate commercialisées notamment sous les dénominations X22-4914, X21-5034 et X21-5037 par la société SHIN-ETSU.
Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.
Parmi les silicones organomodifiées, on peut citer les polyorganosiloxanes comportant :

- des groupements polyéthylèneoxy et/ou polypropylèneoxy comportant éventuellement des groupements alkyle en $C_6$-$C_{24}$ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl ($C_{12}$) méthicone copolyol commercialisée par la société DOW CORNING sous la dénomination Q2 5200 ;
- des groupements aminés substitués ou non comme les produits commercialisés sous la dénomination GP 4 Silicone

Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en $C_1$-$C_4$ ;

- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT;
- des groupements hydroxylés comme les polyorganosiloxanes à fonction hydroxyalkyle décrits dans la demande de brevet français FR-A-85 16334 répondant à la formule (XI) :

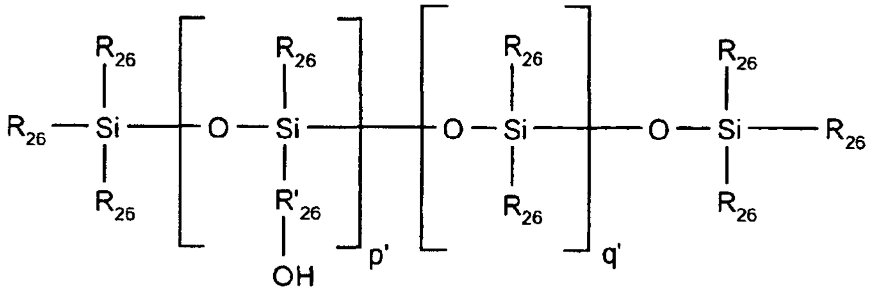

(XI)

dans laquelle les radicaux $R_{26}$ identiques ou différents sont choisis parmi les radicaux méthyle et phényle ; au moins 60 % en mole des radicaux $R_{26}$ désignant méthyle ; le radical $R'_{26}$ est un chaînon alkylène divalent hydrocarboné en $C_2$-$C_{18}$ ; p' est compris entre 1 et 30 inclus ; q' est compris entre 1 et 150 inclus ;

- des groupements acyloxyalkyle tels que par exemple les polyorganosiloxanes décrits dans le brevet US-A-4957732 et répondant à la formule (XII) :

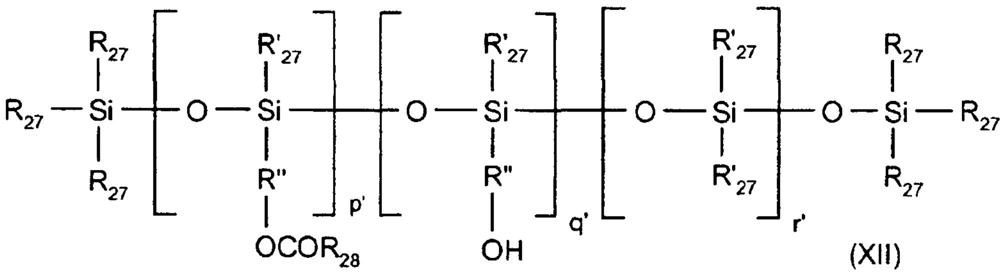

(XII)

dans laquelle :

$R_{27}$ désigne un groupement méthyle, phényle, $-OCOR_{28}$, hydroxyle, un seul des radicaux $R_{27}$ par atome de silicium pouvant être OH ;
$R'_{27}$ désigne méthyle, phényle ; au moins 60 % en proportion molaire de l'ensemble des radicaux $R_4$ et $R'_4$ désignant méthyle ;
$R_{28}$ désigne alkyle ou alcényle en $C_8$-$C_{20}$ ;
R" désigne un radical alkylène hydrocarboné divalent, linéaire ou ramifié en $C_2$-$C_{18}$ ;
r' est compris entre 1 et 120 inclus ;
p' est compris entre 1 et 30 ;
q' est égal à 0 ou est inférieur à 0,5 p', p' + q' étant compris entre 1 et 30 ; les polyorganosiloxanes de formule (XI) peuvent contenir des groupements :

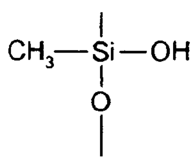

dans des proportions ne dépassant pas 15 % de la somme p' + q' + r'.

- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".

- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par:

a) 50 à 90% en poids d'acrylate de tertiobutyle ;
b) 0 à 40% en poids d'acide acrylique ;
c) 5 à 40% en poids de macromère siliconé de formule :

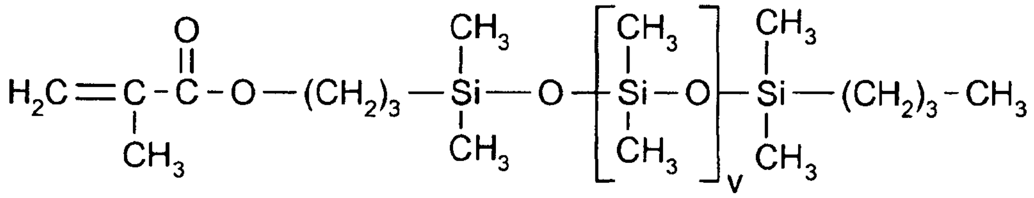

(XIII)

avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions, de nanoémulsions ou de micrémulsions.

Les polyorganosiloxanes particulièrement préférés conformément à l'invention sont :

- les silicones non volatiles choisies dans la famille des polyalkylsiloxanes à groupements terminaux triméthylsilyle telles que les huiles ayant une viscosité comprise entre 0,2 et 2,5 $m^2/s$ à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHODIA CHIMIE, les polyalkylsiloxanes à groupements terminaux diméthylsilanol tels que les diméthiconol ou les polyalkylarylsiloxanes tels que l'huile SILBIONE 70641 V 200 commercialisée par la société RHODIA CHIMIE ;
- la résine d'organopolysiloxane commercialisée sous la dénomination DOW CORNING 593 ;
- les polysiloxanes à groupements aminés tels que les amodiméthicones ou les triméthylsilylamodiméthicone ;

Selon la présente invention, Les composés de type céramide sont notamment les céramides et/ou les glycocéramides et/ou les pseudocéramides et/ou les néocéramides, naturelles ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131.

Des composés de type céramides particulièrement préférés selon l'invention sont par exemple :

- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl)malonamide),
- le N-(2-hydroxyéthyl)-N-(3-cétyloxy-2-hydroxypropyl)amide d'acide cétylique .
- le N-docosanoyl N-méthyl-D-glucamine

ou les mélanges de ces composés.

On peut également utiliser des tensioactifs cationiques parmi lesquels on peut citer en particulier : les sels d'amines grasses primaires, secondaires ou tertiaires, éventuellement polyoxyalkylénées ; les sels d'ammonium quaternaire ; les dérivés d'imidazoline ; ou les oxydes d'amines à caractère cationique.

Les sels d'ammonium quaternaires sont par exemple :

- ceux qui présentent la formule générale (XIV) suivante :

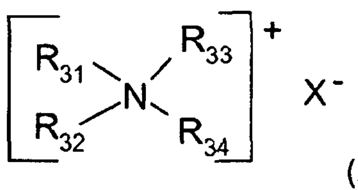

(XIV)

dans laquelle les radicaux $R_{31}$ à $R_{34}$, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène($C_2$-$C_6$), alkylamide, alkyl($C_{12}$-$C_{22}$)amido alkyle($C_2$-$C_6$), alkyl($C_{12}$-$C_{22}$)acétate, hydroxyalkyle, comportant environ de 1 à 30 atomes de carbone; X est un anion choisi dans le groupe des halogénures, phosphates, acétates, lactates, alkyl($C_2$-$C_6$)sulfates, alkyl- ou -alkylarylsulfonates,

- les sels d'ammonium quaternaire de l'imidazolinium, comme par exemple celui de formule (XV) suivante :

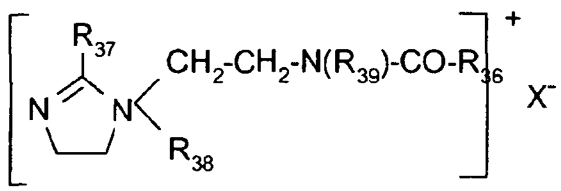

(XV)

dans laquelle $R_{36}$ représente un radical alcényle ou alkyle comportant de 8 à 30 atomes de carbone par exemple dérivés des acides gras du suif, $R_{37}$ représente un atome d'hydrogène, un radical alkyle en $C_1$-$C_4$ ou un radical alcényle ou alkyle comportant de 8 à 30 atomes de carbone, $R_{38}$ représente un radical alkyle en $C_1$-$C_4$, $R_{39}$ représente un atome d'hydrogène, un radical alkyle en $C_1$-$C_4$, X est un anion choisi dans le groupe des halogénures, phosphates, acétates, lactates, alkylsulfates, alkyl-ou-alkylarylsulfonates. De préférence, $R_{36}$ et $R_{37}$ désignent un mélange de radicaux alcényle ou alkyle comportant de 12 à 21 atomes de carbone par exemple dérivés des acides gras du suif, $R_{38}$ désigne méthyle, $R_{39}$ désigne hydrogène. Un tel produit est par exemple commercialisé sous la dénomination «REWOQUAT W 75» par la société DEGUSSA,

- les sels de diammonium quaternaire de formule (XVI) :

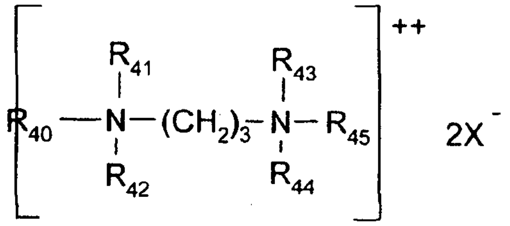

(XVI)

dans laquelle $R_{40}$ désigne un radical aliphatique comportant environ de 16 à 30 atomes de carbone, $R_{41}$, $R_{42}$, $R_{43}$, $R_{44}$ et $R_{45}$, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.

- les sels d'ammonium quaternaire contenant au moins une fonction ester

Les sels d'ammonium quaternaire contenant au moins une fonction ester utilisables selon l'invention sont par exemple ceux de formule (XVII) suivante :

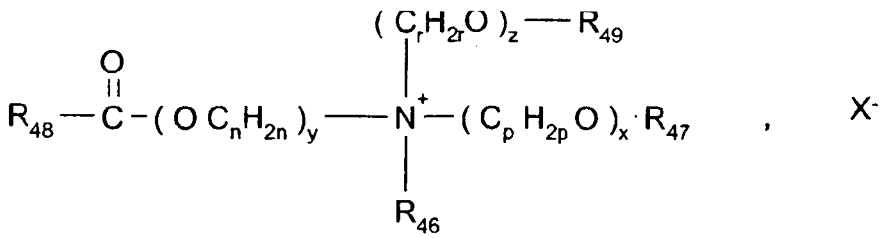

(XVII)

dans laquelle :

- $R_{46}$ est choisi parmi les radicaux alkyles en $C_1$-$C_6$ et les radicaux hydroxyalkyles ou dihydroxyalkyles en $C_1$-$C_6$ ;
- $R_{47}$ est choisi parmi :
  - le radical

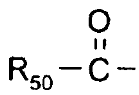

  - les radicaux $R_{51}$ hydrocarbonés en $C_1$-$C_{22}$ linéaires ou ramifiés, saturés ou insaturés,
  - l'atome d'hydrogène,
- $R_{49}$ est choisi parmi :
  - le radical



  - les radicaux $R_{53}$ hydrocarbonés en $C_1$-$C_6$ linéaires ou ramifiés, saturés ou insaturés,
  - l'atome d'hydrogène,
- $R_{48}$, $R_{50}$ et $R_{52}$, identiques ou différents, sont choisis parmi les radicaux hydrocarbonés en $C_7$-$C_{21}$, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;

- X- est un anion simple ou complexe, organique ou inorganique ;

sous réserve que la somme x + y + z vaut de 1 à 15 , que lorsque x vaut 0 alors $R_{47}$ désigne $R_{51}$ et que lorsque z vaut 0 alors $R_{49}$ désigne $R_{53}$.

Les radicaux alkyles $R_{46}$ peuvent être linéaires ou ramifiés et plus particulièrement linéaires.

De préférence $R_{46}$ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque $R_{47}$ est un radical $R_{51}$ hydrocarboné, il peut être long et avoir de 12 à 22 atomes de carbone ou court et avoir de 1 à 3 atomes de carbone.

Lorsque $R_{49}$ est un radical $R_{53}$ hydrocarboné, il a de préférence 1 à 3 atomes de carbone.

Avantageusement, $R_{48}$, $R_{50}$ et $R_{52}$, identiques ou différents, sont choisis parmi les radicaux hydrocarbonés en $C_{11}$-$C_{21}$, linéaires ou ramifiés, saturés ou insaturés, et plus particulièrement parmi les radicaux alkyle et alcényle en $C_{11}$-$C_{21}$, linéaires ou ramifiés, saturés ou insaturés.

De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate.

On utilise plus particulièrement les sels d'ammonium de formule (XVII) dans laquelle :

- $R_{46}$ désigne un radical méthyle ou éthyle,
- x et y sont égaux à 1 ;
- z est égal à 0 ou 1 ;
- n, p et r sont égaux à 2 ;
- $R_{47}$ est choisi parmi :

    - le radical

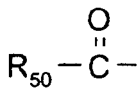

- les radicaux méthyle, éthyle ou hydrocarbonés en $C_{14}$-$C_{22}$

    - l'atome d'hydrogène ;

- $R_{49}$ est choisi parmi :

    - le radical

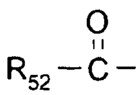

    - l'atome d'hydrogène ;

$R_{48}$, $R_{50}$ et $R_{52}$, identiques ou différents, sont choisis parmi les radicaux hydrocarbonés en $C_{13}$-$C_{17}$, linéaires ou ramifiés, saturés ou insaturés et de préférence parmi les radicaux alkyles et alcényle en $C_{13}$-$C_{17}$, linéaires ou ramifiés, saturés ou insaturés.
Avantageusement, les radicaux hydrocarbonés sont linéaires.

On peut citer par exemple les composés de formule (XVII) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus

particulièrement d'une huile végétale comme l'huile de palme ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société COGNIS, STEPANQUAT par la société STEPAN, NOXAMIUM par la société CECA, REWOQUAT WE 18 par la société DEGUSSA.

On peut aussi utiliser les sels d'ammonium contenant au moins une fonction ester décrits dans les brevets US-A-4874554 et US-A-4137180.

Parmi les sels d'ammonium quaternaire de formule (XIV) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK.

Les acides gras saturés sont choisis plus particulièrement parmi l'acide myristique, l'acide palmitique, l'acide stéarique, l'acide béhénique, et l'acide isostéarique.

Les esters d'acides gras autres que les huiles végétales sont notamment les esters d'acides carboxyliques en particulier les esters mono, di, tri ou tétracarboxyliques.

Les esters d'acides monocarboxyliques sont notamment les monoesters d'acides aliphatiques saturés ou insaturés, linéaires ou ramifiés en $C_1$-$C_{26}$ et d'alcools aliphatiques saturés ou insaturés, linéaires ou ramifiés en $C_1$-$C_{26}$, le nombre total de carbone des esters étant supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en $C_{12}$-$C_{15}$ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle, l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle, le néopentanoate d'isostéaryle, le néopentanoate d'isodécyle.

On peut également utiliser les esters d'acides di ou tricarboxyliques en C4-C22 et d'alcools en $C_1$-$C_{22}$ et les esters d'acides mono di ou tricarboxyliques et d'alcools di, tri, tétra ou pentahydroxy en $C_2$-$C_{26}$.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle.

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle, le néopentanoate d'isostéaryle, le néopentanoate d'isodécyle.

Les huiles fluorées sont par exemple les perfluoropolyéthers décrits notamment dans la demande de brevet EP-A-486135 et les composés fluorohydrocarbonées décrites notamment dans la demande de brevet WO 93/11103.

Le terme de composés fluorohydrocarbonés désigne des composés dont la structure chimique comporte un squelette carboné dont certains atomes d'hydrogène ont été substitués par des atomes de fluor.

Les huiles fluorées peuvent également être des fluorocarbures tels que des fluoramines par exemple la perfluorotributylamine, des hydrocarbures fluorés, par exemple le perfluorodécahydronaphtalène, des fluoroesters et des fluoroethers ;

Les perfluoropolyéthers sont par exemple vendus sous les dénominations commerciales FOMBLIN par la

société MONTEFLUOS et KRYTOX par la société DU PONT.

Parmi les composés fluorohydrocarbonés, on peut également citer les esters d'acides gras fluorés tels que le produit vendu sous la dénomination NOFABLE FO par la société NIPPON OIL.

Il est bien entendu possible de mettre en oeuvre des mélanges d'agents conditionneurs.

Les agents conditionneurs préférés selon l'invention sont les polymères cationiques, les tensioactifs cationiques et les silicones et leurs mélanges.

Selon l'invention, le ou les agents conditionneurs peuvent représenter de 0,001 % à 20 % en poids, de préférence de 0,01 % à 10% en poids et plus particulièrement de 0,1 à 3% en poids par rapport au poids total de la composition finale.

Les compositions protectrices de la couleur des fibres kératiniques selon l'invention peuvent se présenter sous forme de lotions aqueuses ou hydroalcooliques. Les compositions cosmétiques selon l'invention peuvent également se présenter sous forme d'huile, de gel, de lait, de crème, d'émulsion ou de mousse.

Ces compositions peuvent également être anhydres, c'est-à-dire contenir moins de 5% d'eau.

Les compositions protectrices de la couleur des fibres kératiniques peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Le pH de la composition protectrice de la couleur des fibres kératiniques varie généralement de 1 à 11. Il est de préférence de 2 à 6 pour les produits non colorants, et peut être ajusté à la valeur désirée au moyen d'agents acidifiants ou alcalinisants bien connus de l'état de la technique des compositions appliquées sur des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les éthylènediamines oxyéthylénées et/ou oxypropylénées, les hydroxydes de sodium ou de potassium et les composés de formule suivante :

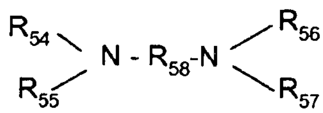

dans laquelle $R_{58}$ est un reste propylène éventuellement substitué par un groupement hydroxyle ou un radical alkyle en $C_1$-$C_4$ ; $R_{54}$, $R_{55}$, $R_{56}$ et $R_{57}$, identiques ou différents, représentent un atome d'hydrogène, un radical alkyle en $C_1$-$C_4$ ou hydroxyalkyle en $C_1$-$C_4$.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

L'invention a aussi pour objet un procédé pour protéger vis-à-vis du lavage la couleur des fibres kératiniques teintes artificiellement, caractérisé par le fait qu'il consiste à appliquer sur lesdites fibres, de préférence avant ou après teinture, une ou plusieurs huiles siccatives ou au moins une composition comprenant dans un milieu cosmétiquement acceptable une ou plusieurs huiles siccatives.

De manière préférentielle, la composition comprenant la ou les huiles siccatives sera appliquée sur lesdites fibres après l'étape de teinture.

Il est aussi possible de prévoir, une étape de rinçage et/ou une étape de lavage au shampoing avant ou après l'application de la composition contenant la ou les huiles siccatives.

Le procédé selon l'invention peut comprendre une étape supplémentaire de séchage total ou partiel des fibres kératiniques.

Selon une forme particulière de l'invention, le procédé de protection de la couleur des fibres kératiniques peut comprendre une étape de chauffage de la composition comprenant la ou les huiles siccatives que l'on appliquera ensuite directement sur les fibres kératiniques. La température sera de préférence inférieure ou égale à 120°C.

Selon une forme particulière de l'invention, le procédé de protection de la couleur des fibres kératiniques peut comprendre une étape de chauffage des fibres kératiniques pendant ou après application de la composition comprenant la ou les huiles siccatives.

Le chauffage des fibres kératiniques peut par exemple être effectué au moyen d'un fer, d'un mélange eau liquide/vapeur d'eau ou bien au moyen d'un casque chauffant.

Le fer chauffant utile dans le cadre de l'invention est un fer chauffant conventionnellement utilisé dans le domaine capillaire. Un tel fer, par exemple un fer à friser ou un fer à lisser, est bien connu dans le domaine du traitement capillaire. Par exemple, des fers utiles pour mettre en oeuvre la présente invention sont des fers plats ou ronds décrits dans les brevets US 4 103 145, US 4 308 878, US 5 983 903, US 5 957 140, US 5 494 058. L'application du fer peut

se faire par touches séparées successives de quelques secondes, ou par déplacement ou glissement progressif le long des mèches. Il est possible entre l'application de la composition protectrice de la couleur et l'application du fer chauffant sur les fibres kératiniques de prévoir un temps de pause. Ledit temps de pause de préférence variera de 30 secondes à 60 minutes et plus préférentiellement de 1 à 30 minutes. La température va de préférence de 60°C à 120°C.

Le mélange eau liquide/vapeur d'eau utile dans le cadre de l'invention a en général une température d'au moins 35°C.

Le mélange eau liquide/vapeur d'eau constitue une brume. Ledit mélange peut contenir en plus au moins un autre gaz tel que l'oxygène ou l'azote, des mélanges de gaz tels que l'air ou bien encore d'autres composés vaporisables.

La température du mélange eau liquide/vapeur d'eau est de préférence supérieure ou égale à 40°C, et est plus particulièrement comprise entre 40°C et 75°C environ.

De préférence, le mélange eau liquide/vapeur d'eau est mis en contact avec la fibre pendant une durée allant de 1 seconde à 1 heure, et encore plus préférentiellement de 5 minutes à 15 minutes. Bien entendu, l'application dudit mélange peut être répétée plusieurs fois sur la même fibre, chaque opération se faisant selon une durée telle qu'indiquée ci-dessus. Plus préférentiellement, on applique tout d'abord sur les cheveux la composition contenant composés à base de zinc puis on soumet ces mèches ainsi imprégnées à l'action du mélange eau liquide/vapeur d'eau selon les conditions mentionnées précédemment, puis on refroidit les mèches ainsi traitées par exemple en envoyant sur ou à travers celles-ci un courant d'air froid ou d'air à température ambiante.

La production du mélange eau liquide/vapeur d'eau utilisé selon l'invention peut se faire à l'aide de tout appareil connu en soi et prévu à cet effet. Toutefois, selon la présente invention, on utilise de préférence un appareil comprenant au moins un générateur de vapeur d'eau directement relié à un casque diffusant sur les fibres kératiniques en particulier les cheveux humains le mélange eau liquide/vapeur d'eau. Comme type d'appareil, on utilisera plus particulièrement celui vendu sous le nom ®MICROMIST par la Société TAKARA BELMONT.

Un autre objet de l'invention concerne un procédé de coloration consistant à appliquer sur les fibres kératiniques humaines et plus particulièrement des cheveux, une composition (A) colorante directe ou bien une composition colorante (A) d'oxydation en présence le cas échéant d'un ou plusieurs agents oxydants, pendant un temps suffisant pour développer la couleur, et de faire précéder ou suivre cette application par l'application d'une composition (B) contenant dans un milieu cosmétiquement acceptable une ou plusieurs huiles siccatives telles que définies précédemment.

L'application de la composition (A) peut être suivie d'un rinçage et/ou d'un séchage des fibres kératiniques.

L'application de la composition (B) peut être suivie d'un rinçage et/ou d'un séchage des fibres kératiniques. La composition (B) peut être préalablement chauffée dans les mêmes conditions définies ci-dessus. L'application de la composition (B) peut être suivie par un chauffage des fibres kératiniques dans les mêmes conditions définies ci-dessus.

De manière préférentielle, la composition (B) sera appliquée après l'application de la composition (A) colorante directe ou d'oxydation. La composition (B) comprenant la ou les huiles siccatives peut être appliquée immédiatement après coloration ou de manière différée. Par différée, on entend une application se faisant quelques heures, un jour ou plusieurs jours (de 1 à 15 jours) après la coloration. De préférence, la composition (B) sera appliquée immédiatement après coloration des fibres kératiniques. ; les applications de ladite composition pouvant être répétées entre deux colorations.

Dans le cas des colorations directes éclaircissantes les compositions colorantes (A) résultent du mélange au moment de l'emploi d'une composition colorante ($A_1$) contenant un ou plusieurs colorants directs et d'une composition ($A_2$) contenant un ou plusieurs agents oxydants.

Dans le cas des colorations d'oxydation, les compositions colorantes (A) résultent du mélange au moment de l'emploi d'une composition colorante ($A_3$) contenant une ou plusieurs bases d'oxydation et éventuellement un ou plusieurs coupleurs et/ou un ou plusieurs colorants directs et d'une composition ($A_4$) contenant un ou plusieurs agents oxydants.

Un autre objet de l'invention concerne un procédé de coloration consistant à appliquer sur les fibres kératiniques en particulier les fibres kératiniques humaines et plus particulièrement les cheveux, une composition (A) colorante directe pendant un temps suffisant pour développer la couleur, ladite composition (A) comprenant dans un milieu cosmétiquement acceptable une ou plusieurs huiles siccatives, choisies parmi l'huile de lin.

Les colorants directs sont plus particulièrement des composés absorbant les radiations lumineuses dans le domaine visible (400-750 nm). Ils peuvent être de nature non ionique, anionique ou cationique.

Généralement, les colorants directs sont choisis parmi les colorants benzéniques nitrés, les colorants azoïques, anthraquinoniques, naphtoquinoniques, benzoquinoniques, phénotiaziniques, indigoïdes, xanthéniques, phénanthridiniques, phtalocyanines, ceux dérivés du triarylméthane, seuls ou en mélanges.

Parmi les colorants benzéniques nitrés, on peut citer les composés rouges ou orangés suivants : le 1-hydroxy-3-nitro-4-N-(γ-hydroxypropyl)amino benzène, le N-(β-hydroxy éthyl)amino-3-nitro-4-amino benzène, le 1-amino-3-méthyl-4-N-(β-hydroxyéthyl)amino-6-nitro benzène, le 1-hydroxy-3-nitro-4-N-(β-hydroxyéthyl)-amino benzène, le 1,4-diamino-2-nitrobenzène, le 1-amino-2-nitro-4-méthylamino benzène, la N-(β-hydroxyéthyl)-2-nitro-paraphénylènediamine, le 1-amino-2-nitro-4-(β-hydroxy éthyl)amino-5-chloro benzène, la 2-nitro-4-amino-diphénylamine, le 1-amino-3-nitro-6-hydroxybenzène, le 1-(β-amino éthyl)amino-2-nitro-4-(β-hydroxyéthyloxy) benzène, le 1-(β, γ-dihydroxypropyl)oxy-3-nitro-4-(β-hydroxyéthyl)amino benzène, le 1-hydroxy-3-nitro-4-aminobenzène, le 1-hydroxy-2-amino-4,6-dinitrobenzène, le 1-méthoxy-3-nitro-4-(β-hydroxyéthyl)amino benzène, la 2-nitro-4'-hydroxydiphénylamine, le 1-amino-2-nitro-4-hydroxy-5-méthylbenzène, seuls ou en mélanges.

En ce qui concerne les colorants directs benzéniques nitrés, on peut mettre en oeuvre des colorants de ce type jaunes et jaune-verts, comme par exemple le 1-β-hydroxyéthyloxy-3-méthylamino-4-nitrobenzène, le 1-méthylamino-2-nitro-5-(β, γ-dihydroxypropyl)oxy benzène, le 1-(β-hydroxyéthyl)amino-2-méthoxy-4-nitrobenzène, le 1-(β-amino éthyl)amino-2-nitro-5-méthoxy-benzène, le 1,3-di(β-hydroxyéthyl)amino-4-nitro-6-chloro benzène, le 1-amino-2-nitro-6-méthyl-benzène, le 1-(β-hydroxyéthyl)amino-2-hydroxy-4-nitrobenzène, la N-(β-hydroxyéthyl)-2-nitro-4-trifluorométhylaniline, l'acide 4-(β-hydroxyéthyl)amino-3-nitro-benzènesulfonique, l'acide 4-éthylamino-3-nitro-benzoïque, le 4-(β-hydroxyéthyl)amino-3-nitro-chlorobenzène, le 4-(β-hydroxyéthyl)amino-3-nitro-méthyl benzène, le 4-(β, γ-dihydroxypropyl)amino-3-nitro-trifluorométhylbenzène, le 1-(β-uréido éthyl)amino-4-nitrobenzène, le 1,3-diamino-4-nitrobenzène, le 1-hydroxy-2-amino-5-nitrobenzène, le 1-amino-2-[tris(hydroxyméthyl)méthyl]amino-5-nitro-benzène, le 1-(β-hydroxyéthyl)amino-2-nitrobenzène, le 4-(β-hydroxyéthyl)amino-3-nitrobenzamide.

Il est de même envisageable d'utiliser des colorants benzéniques nitrés bleus ou violets, comme entre autres le 1-(β-hydroxyéthyl)amino-4-N,N-bis-(β-hydroxyéthyl)amino 2-nitrobenzène, le 1-(γ-hydroxypropyl)amino 4-N,N-bis-(β-hydroxyéthyl)amino 2-nitrobenzène, le 1-(β-hydroxyéthyl)amino 4-(N-méthyl, N-β-hydroxyéthyl)amino 2-nitrobenzène, le 1-(β-hydroxyéthyl)amino 4-(N-éthyl, N-β-hydroxyéthyl)amino 2-nitrobenzène, le 1-(β, γ-dihydroxypropyl)amino 4-(N-éthyl, N-β-hydroxyéthyl)amino 2-nitrobenzène, les 2-nitroparaphénylènediamines de formule suivante :

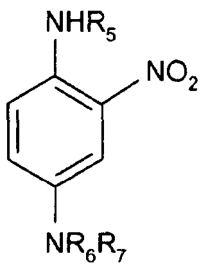

dans laquelle :

- $R_6$ représente un radical alkyle en C1-C4, un radical β-hydroxyéthyle ou β-hydroxypropyle ou γ-hydroxypropyle ;

$R_5$ et $R_7$, identiques ou différents, représentent un radical β-hydroxyéthyle, β-hydroxypropyle, γ-hydroxypropyle, ou β, γ-dihydroxypropyle, l'un au moins des radicaux $R_6$, $R_7$ ou $R_5$ représentant un radical γ-hydroxypropyle et $R_6$ et $R_7$ ne pouvant désigner simultanément un radical β-hydroxyéthyle lorsque $R_6$ est un radical γ-hydroxypropyle, telles que celles décrits dans le brevet français FR 2 692 572.

Il est rappelé que les colorants azoïques sont des composés comportant dans leur structure au moins un enchaînement -N=N- non inclus dans un cycle ; les colorants méthiniques sont des composés comportant dans leur structure au moins un enchaînement -C=C- non inclus dans un cycle ; les colorants azométhinique sont des composés comportant dans leur structure au moins un enchaînement -C=N- non inclus dans un cycle.

Les colorants dérivés de triarylméthane comportent dans leur structure au moins un enchaînement suivant :

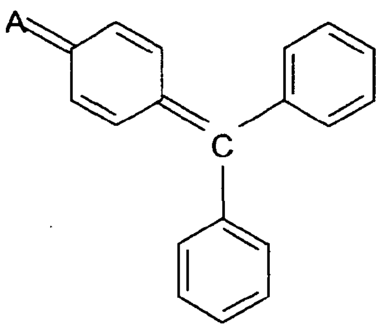

A désignant un atome d'oxygène ou d'azote

Les colorants xanthéniques comportent dans leur structure au moins un enchaînement de formule :

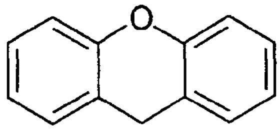

Les colorants phénanthridiniques comportent dans leur structure au moins un enchaînement de formule

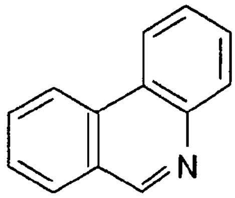

Les colorants phtalocyanines comportent dans leur structure au moins un

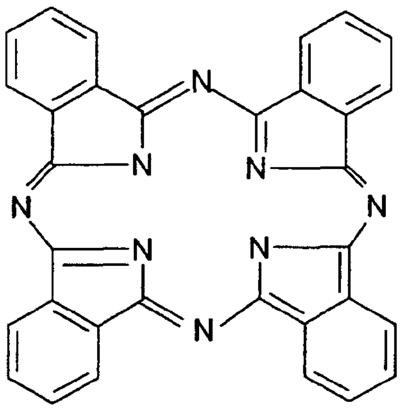

enchaînement de formule :

Les colorants phénotiaziniques comportent dans leur structure au moins un enchaînement suivant :

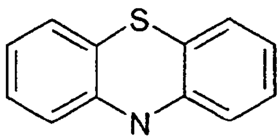

Les colorants directs peuvent de plus être choisis parmi les colorants basiques comme ceux listés dans le Color Index, 3ème édition, notamment sous les dénominations "Basic Brown 16", "Basic Brown 17", "Basic Yellow 57", "Basic Red 76", "Basic Violet 10", "Basic Blue 26" et "Basic Blue 99" ; ou parmi les colorants directs acides, listés le Color Index, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore les colorants directs cationiques tels que ceux décrits dans les demandes de brevet WO 95/01772, WO 95/15144 et EP 714954 et en particulier « Basic Red 51 », « Basic Orange 31 », « Basic Yellow 87 ».

Lorsqu'ils sont présents, le ou les colorants directs représentent de préférence de 0,0005 à 12 % en poids environ du poids total de la composition tinctoriale, et encore plus préférentiellement de 0,005 à 6 % en poids environ de ce poids.

Les bases d'oxydation peuvent être choisies parmi les bases d'oxydation classiquement utilisées en teinture d'oxydation et parmi lesquelles on peut notamment citer les paraphénylènediamines , les bis-phénylalkylènediamines, les paraaminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl

paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl)paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,y-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl)éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl)éthylènediamine, le 1,8-bis-(2,5-diamino phénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl)phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]-pyrimidin-7-yl)-(2-hydroxy-éthyl)-amino]-éthanol, le 2-[(7-amino-pyrazolo[1,5-a]-pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2,5, N7,N7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl)pyrazole, le 3,4-diamino pyrazole, le 4,5-diamino 1-(4'-chlorobenzyl)pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl)pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

A titre de dérivés pyrazoliques, on peut aussi citer les diamino-N,N-dihydropyrazolopyrazolones et notamment celles décrites dans la demande FR 2 886 136 telles que les composés suivants et leurs sels d'addition.

Parmi ces composés, les préférés sont les :

2,3-Diamino-6,7-dihydro-1H,5H-pyrazolo[1,2-a]pyrazol-1-one,
2-Amino-3-éthylamino-6,7-dihydro-1H,5H-pyrazolo[1,2-a]pyrazol-1-one,
2-Amino-3-isopropylamino-6,7-dihydro-1H,5H-pyrazolo[1,2-a]pyrazol-1-one,
2-Amino-3-(pyrrolidin-1-yl)-6,7-dihydro-1H,5H-pyrazolo[1,2-a]pyrazol-1-one,
4,5-diamino-1,2-diméthyl-1,2-dihydro-pyrazol-3-one,
4,5-diamino-1,2-diéthyl-1,2-dihydro-pyrazol-3-one,
4,5-diamino-1,2-di-(2-hydroxyéthyl)-1,2-dihydro-pyrazol-3-one,
2-amino-3-(2-hydroxyéthyl)amino-6,7-dihydro-1H,5H-pyrazolo[1,2-a]pyrazol-1-one,
2-amino-3-diméthylamino-6,7-dihydro-1H,5H-pyrazolo[1,2-a]pyrazol-1-one,
2,3-diamino-5,6,7,8-tétrahydro-1H,6H-pyridazino[1,2-a]pyrazol-1-one,
4-Amino-1,2-diethyl-5-(pyrrolidin-1-yl)-1,2-dihydro-pyrazol-3-one,
4-Amino-5-(3-dimethylamino-pyrrolidin-1-yl)-1,2-diethyl-1,2-dihydro-pyrazol-3-one,
2,3-diamino-6-hydroxy-6,7-dihydro-1H,5H-pyrazolo[1,2-a]pyrazol-1-one,

A titre de bases hétérocycliques ou d'autres colorants d'oxydation différents de la 3-amino 2-méthylamino 6-méthoxypyridine ou un de ses sels d'addition avec un acide, on utilisera préférentiellement le 4,5-diamino 1-(β-hydroxyéthyl) pyrazole et/ou la 2,3-diamino-6,7-dihydro-1H,5H-pyrazolo[1,2-a]pyrazol-1-one et leurs sels d'addition.

Lorsqu'elles sont utilisées, ces bases d'oxydation représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols, les naphtols et les coupleurs hétérocycliques tels que par exemple les dérivés indoliques, les dérivés indoliniques, les dérivés pyridiniques, les dérivés indazoliques, les dérivés de pyrazolo[1,5-b]-1,2,4-triazole, les dérivés de pyrazolo[3,2-c]-1,2,4-triazole, les dérivés de benzimidazole, les dérivés de benzothiazole, les dérivés de benzoxazole, les dérivés de 1,3-benzodioxole et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy)benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 2-méthyl-1-naphtol, le 6-hydroxy indole, le 4-hydroxy indole, le 4-hydroxy N-méthyl indole, la 6-hydroxy indoline, la 6-hydroxybenzomorpholine, la 3,5 diamino 2,6-diméthoxy pyridine, le 1-N-(β hydroxyéthyl)amino 3,4 méthylènedioxy benzène, le 2,6 bis(β hydroxyéthylamino)toluène, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents conditionneurs tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'agent oxydant, utilisé dans la coloration directe éclaircissante (coloration directe avec un agent oxydant) ou dans la coloration d'oxydation, est de préférence choisi dans le groupe formé par le peroxyde d'hydrogène, le peroxyde d'urée, les bromates ou ferricyanures de métaux alcalins, les persels tels que les perborates et les persulfates. On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif. Le peroxyde d'hydrogène est préféré.

L'invention a également pour objet un agent de coloration multi-composants (ou multi-compartiments) ou kit comportant au moins un premier composant (ou compartiment) comprenant une composition (A) contenant dans un milieu aqueux cosmétiquement acceptable, une ou plusieurs huiles siccatives et au moins un colorant direct, associé à un deuxième composant (ou compartiment) comprenant un ou plusieurs agents oxydants, dans lequel l'huile riccative est choisie parmi l'huile de lin.

L'invention va être maintenant plus complètement illustrée à l'aide des exemples suivants qui ne sauraient être considérés comme limitatifs.

**EXEMPLES :**

Les compositions selon l'invention utilisables en protection de la coloration peuvent être exemplifiées par la liste non exhaustive suivante :

Les concentrations sauf mention contraire sont en matières actives.

**EXEMPLE 1 : Huile :**

On a préparé une huile à appliquer avant un shampooing de composition suivante :

| | |
|---|---|
| Huile de graines de lin partiellement polymérisée (STANDOLIE DE LIN 60P de NOVANCE) | 30 g |
| Acide salicylique | 0,1 g |
| Conservateur | 0,1 g |
| Huile de vaseline qs | 100 g |

**EXEMPLE 2 : shampooing**

On a préparé les shampoings suivants :

| | A | B |
|---|---|---|
| Chlorure de sodium | 2,3 g | 2,3 g |
| Huile de graines de lin partiellement polymérisée (STANDOLIE DE LIN 60P de NOVANCE) | 1 g | - |
| Acide salicylique | 0,2 g | 0,2 g |
| Conservateurs | qs | qs |
| Chlorure de poly di-methyl di-allyl ammonium dans l'eau a 40 % (MERQUAT 100 de NALCO) | 0,4 g | 0,4 g |
| Lauryl éther sulfate de sodium (à 2.2 moles d'oxyde d'éthylène) en solution aqueuse à 70% MA | 4,9 g | 4,9 g |
| Cocoyl amidopropyl betaine en solution aqueuse à 30% de MA | 6,9 g | 6,9 g |
| Oléate de propylène glycol polyéthoxylé (55 oe) et de propylène glycol en solution hydroglycolique (ANTIL 141 LIQUID de EVONIK GOLDSCHMIDT) | 0,48 g | 0,48 g |
| Hexylène Glycol | 1 g | 1 g |
| Agent de PH (NaOH ou acide citrique) qs PH | 5,3 | 5,3 |
| Eau désionisée QS | 100 g | 100 g |

Démonstration de l'effet de protection de la couleur vis-à-vis du lavage

Etape de coloration:

On applique un mélange de la composition du tableau 1 ci-après et d'eau oxygénée (Eau oxygénée l'Oréal professionnel 20 volumes à 6%) poids pour poids sur des mèches de cheveux 90% blancs permanentées en quantité suffisante pour bien imprégner les mèches (dans ce cas 10 g de mélange colorant/ g de mèche). Le temps de pause

est de 15 minutes de chaque côté de la mèche. Les mèches sont alors rincées à l'eau, puis lavées par le shampooing DOP camomille et séchées.

Tableau 1

| composition 1 colorante | quantités % |
|---|---|
| Alcool oléique polyglycérolé à 2 moles de glycérol | 4g |
| Alcool oléique polyglycérolé à 4 moles de glycérol (78% M.A) | 5.69g |
| Acide oléique | 3g |
| Amine oléique 2 OE commercialisée sous la dénomination d' ETHOMEEN 012 parla société AKZO7 g | 7g |
| Laurylamino succinamate de diéthylaminopropyle, sel de sodium à 55% M.A. | 3g |
| Alcool oléique | 5g |
| Diéthanolamide d'acide oléique | 12g |
| Propylène glycol | 3.5g |
| Alcool éthylique | 7g |
| Dipropylène glycol | 0.5g |
| Monométhyléther de propylène glycol | 9g |
| Métabisulfite de sodium en solution aqueuse à 35% M.A | 0.455g |
| Acétate d'ammonium | 0.8g |
| Antioxydant, séquestrant | q.s |
| Parfum, conservateur | q.s |
| p -Phénylène diamine | $6.10^{-4}$mol |
| 4-amino-2-hydroxytoluène | $6.10^{-4}$mol |
| Ammoniaque à 20% de $NH_3$ | 10g |
| Eau déminéralisée | q.s.p. 100g |

Etapes de traitement protecteur:

On procède comme suit :

- application de la composition A ou B en quantité suffisante pour bien imprégner les mèches (dans ce cas 2,5 grammes par gramme de cheveu sur les cheveux colorés).
- -5minutes de pose
- Rinçage et séchage au casque.

Etapes de ténacité après lavages aux shampooings:

Les mèches traitées subissent alors une épreuve de ténacité lavages aux shampooings comparativement à des mèches colorées non traitées.

Pour cela, les mèches subissent 8 shampooings successifs avec les compositions A ou B avec séchage intermédiaire.

Evaluation de la protection

La dégradation de la couleur après lavages des mèches traitées et non traitées est évaluée visuellement par rapport à des mèches colorées non lavées.

Un suivi spectro-colorimétrique accompagne ces évaluations. Des mesures sont réalisées à l'aide du spectro-colorimètre MINOLTA CM2022 :

La dégradation provoquée par les lavages est exprimée en ΔE

$$\Delta E(8 \text{ shampooings - 0 shampooing}) = \sqrt{(\Delta L^{*2} + \Delta a^{*2} + \Delta b^{*2})}$$

On exprime alors la protection par une différence en ΔE entre les mèches traitées et non traitées.

Résultats :

Après l'épreuve de ténacité lavages, on observe une dégradation importante de la coloration des mèches colorées non traitées

On observe de façon surprenante, qu'après cette même épreuve, l'utilisation de la composition A de l'invention apporte une protection significative de la couleur par rapport aux mèches non traitées.

Ces résultats sont confirmés par les mesures colorimétriques qui indiquent un gain en ΔE significatif par rapport à la mèche non traitée après 8 shampooings.

*Résultats de protection de la couleur après épreuve de ténacité aux lavages*

| Mèche | *ΔE par rapport aux mèches non lavées* |
|---|---|
| Mèche traitée par la composition B après 8 shampooings | **9,83** |
| Mèche traitée par la composition A selon l'invention après 8 shampooings | **5,72** |

Plus le ΔE est petit, moins il y a de dégradation de la couleur par les lavages. La résistance aux lavages est significativement meilleure avec la composition de l'invention A.

**EXEMPLE 3 : shampooing.**

On a préparé le shampoing suivant :

| | |
|---|---|
| Huile de graines de lin partiellement polymérisée 0,1 (STANDOLIE DE LIN 60P de NOVANCE) | g |
| Acide salicylique | 0,2 g |
| Conservateurs | qs |
| Chlorure de poly di-methyl di-allyl ammonium dans l'eau à 40 % (MERQUAT 100 de NALCO) | 0,4 g |
| Lauryl éther sulfate de sodium à 2,2 moles d'oxyde d'éthylène en solution aqueuse à 70% MA | 4 g |
| Cocoyl amidopropyl bétaine en solution aqueuse à 30% MA | de 4,4 g |
| Alkyl (C8/C16) polyglucoside (1.4) en solution aqueuse à 53 % de MA | 5 g |
| Acide lauryl éther carboxylique (4.5 OE) (EMPICOL CED / FL de HUNTSMAN) | 5 2,97 g |
| Propylène glycol | 2 g |
| Parfum | 0,5 g |
| | Qs pH |
| Agent de pH | 5.3 |
| Eau désionisée qs | 100 g |

Ce shampooing protège la couleur des cheveux colorés artificiellement.

**EXEMPLE 4 : Après-shampooing**

On a préparé l'après-shampoing suivant :

| | |
|---|---|
| Alcool cétylstéarylique (C16/C18 50/50) | 2,5 g |
| Mélange myristate/palmitate/stéarate de myristyle/cétyle/stéaryle (MIRACETI de LASERSON) | 0,5 g 0,5 g |
| Huile de Palme | 2 g |

(suite)

| | |
|---|---|
| Huile de graines de lin partiellement polymérisée (STANDOLIE DE LIN 60P de NOVANCE) | 1 g |
| Conservateurs | 0,33 g |
| Alcool cétylstéarylique oxyéthylene (33 OE) | 0,8 g |
| Parfum | 0,4 g |
| Phosphate de diamidon de mais hydroxypropylé prégélatinisé en solution aqueuse à 90%MA | 4,5 g |
| (STRUCTURE ZEA de AKZO NOBEL) | Qs pH 5.3 |
| Agent de pH | |
| Eau desionisée qs | 100 g |

Cet après-shampooing protège la couleur des cheveux colorés artificiellement.

## Revendications

1. Utilisation d'une ou plusieurs huiles siccatives comme agent permettant de protéger vis-à-vis du lavage la couleur des fibres kératiniques teintes artificiellement, notamment des fibres kératiniques humaines et plus particulièrement des cheveux, dans laquelle les huiles siccatives sont choisies parmi l'huile de lin, l'huile de bois de Chine (ou Canton) appelée aussi huile de tung, l'huile d'oïticica, l'huile de vernonia, l'huile d'oeillette, l'huile de grenade, l'huile de calendula, l'huile de Perilla et leurs mélanges.

2. Utilisation selon la revendication 1, **caractérisée en ce que** les fibres kératiniques sont teintes par coloration d'oxydation, en présence d'un ou plusieurs agents oxydants.

3. Utilisation selon l'une des revendications précédentes, **caractérisée en ce que** l'huile siccative est l'huile de lin.

4. Utilisation selon l'une des revendications précédentes, **caractérisée en ce que** la ou les huiles siccatives sont des huiles raffinées.

5. Utilisation selon l'une des revendications précédentes, **caractérisée en ce que** la ou les huiles siccatives sont partiellement polymérisées par soufflage ou polymérisation à chaud.

6. Utilisation selon la revendication 5, **caractérisée en ce que** la ou les huiles siccatives sont choisies parmi les standolies de lin.

7. Utilisation selon l'une quelconque des revendications précédentes, où la ou les huiles siccatives sont présentes dans les compositions dans des concentrations allant de 0,05 à 100% en poids et plus préférentiellement de 0,1 à 40% en poids et encore plus préférentiellement de 0,1 à 30% mieux de 0,5 à 10 % en poids par rapport au poids total de la composition.

8. Procédé pour protéger la couleur vis-à-vis du lavage des fibres kératiniques teintes artificiellement notamment des fibres kératiniques humaines et plus particulièrement des cheveux, **caractérisé par le fait qu'**il consiste à appliquer sur lesdites fibres, une ou plusieurs huiles siccatives ou une composition comprenant dans un milieu cosmétiquement acceptable une ou plusieurs huiles siccatives ; lesdites huiles siccatives étant choisies parmi l'huile de lin, l'huile de bois de Chine (ou Canton) appelée aussi huile de tung, l'huile d'oïticica, l'huile de vernonia, l'huile d'oeillette, l'huile de grenade, l'huile de calendula, l'huile de Perilla et leurs mélanges.

9. Procédé selon la revendication 8, selon lequel les fibres kératiniques sont teintes par coloration d'oxydation.

10. Procédé de coloration des fibres kératiniques humaines et plus particulièrement des cheveux, consistant à appliquer sur les fibres, une composition (A) colorante directe ou bien une composition (A) d'oxydation éventuellement en présence d'un ou plusieurs agents oxydants pendant un temps suffisant pour développer la couleur, et de faire précéder ou suivre cette application par l'application d'une composition (B) contenant dans un milieu cosmétiquement acceptable une ou plusieurs huiles siccatives choisies parmi l'huile de lin, l'huile de bois de Chine (ou Canton) appelée aussi huile de tung, l'huile d'oïticica, l'huile de vernonia, l'huile d'oeillette, l'huile de grenade, l'huile de calendula, l'huile de Perilla et leurs mélanges.

**11.** Procédé selon la revendication 10, où l'application de la composition (A) est suivie d'un rinçage et/ou d'un séchage des fibres kératiniques.

**12.** Procédé selon l'une des revendications 10 ou 11 où l'application de la composition (B) est suivie d'un rinçage et/ou d'un séchage des fibres kératiniques et/ou d'un chauffage des fibres kératiniques.

**13.** Procédé selon l'une quelconque des revendications 10 à 12 où la composition (B) est appliquée après l'application de la composition (A) colorante directe ou d'oxydation, soit immédiatement, soit en différé, et les applications de ladite composition (B) pouvant être répétées entre deux colorations.

**14.** Procédé selon l'une quelconque des revendications 10 à 13, où la composition colorante (A) résulte du mélange au moment de l'emploi d'une composition colorante ($A_1$) comprenant un ou plusieurs colorants directs et d'une composition ($A_2$) comprenant un ou plusieurs agents oxydants.

**15.** Procédé selon l'une quelconque des revendications 10 à 14, où la composition colorante (A) résulte du mélange au moment de l'emploi d'une composition colorante ($A_3$) comprenant une ou plusieurs bases d'oxydation et éventuellement un ou plusieurs coupleurs et/ou un ou plusieurs colorants directs et d'une composition ($A_4$) comprenant un ou plusieurs agents oxydants.

**16.** Procédé de coloration des fibres kératiniques, en particulier des fibres kératiniques humaines et plus particulièrement des cheveux, consistant à appliquer sur les fibres, une composition (A) colorante directe pendant un temps suffisant pour développer la couleur, ladite composition (A) comprenant dans un milieu cosmétiquement acceptable une ou plusieurs huiles siccatives choisies parmi l'huile de lin.

**17.** Kit comportant au moins un premier composant (ou compartiment) comprenant une composition colorante (A) comprenant dans un milieu cosmétiquement acceptable, une ou plusieurs huiles siccatives choisies parmi l'huile de lin; et au moins un colorant direct, associés à un deuxième composant (ou compartiment) comprenant un ou plusieurs agents oxydants.

**Patentansprüche**

**1.** Verwendung von einem oder mehreren die Trocknung beschleunigenden Ölen als Mittel zum Schutz der Farbe künstlich gefärbter Keratinfasern, insbesondere menschlicher Keratinfasern und ganz besonders der Haare, gegenüber Wäsche, wobei die die Trocknung beschleunigenden Öle ausgewählt sind aus Leinöl, chinesischem Holzöl, auch als Tungöl bezeichnet, Oiticicaöl, Vernoniaöl, Schlafmohnöl, Granatapfelkernöl, Calendulaöl, Perillaöl und deren Gemischen.

**2.** Verwendung nach Anspruch 1, **dadurch gekennzeichnet, dass** die Keratinfasern mittels Oxidationsfärbung in Gegenwart von einem oder mehreren Oxidationsmitteln, gefärbt werden.

**3.** Verwendung nach einem der vorhergehenden Ansprüche, **dadurch gekennzeichnet, dass** es sich bei dem die Trocknung beschleunigenden Öl um Leinöl handelt.

**4.** Verwendung nach einem der vorhergehenden Ansprüche, **dadurch gekennzeichnet, dass** es sich bei dem oder den die Trocknung beschleunigende(n) Öl(en) um raffinierte Öle handelt.

**5.** Verwendung nach einem der vorhergehenden Ansprüche, **dadurch gekennzeichnet, dass** das oder die die Trocknung beschleunigende(n) Öl(e) durch das Blasverfahren oder Heißpolymerisation partiell polymerisiert sind.

**6.** Verwendung nach Anspruch 5, **dadurch gekennzeichnet, dass** das oder die die Trocknung beschleunigende(n) Öl(e) aus Lein-Standölen ausgewählt ist/sind.

**7.** Verwendung nach einem der vorhergehenden Ansprüche, wobei das oder die die Trocknung beschleunigende(n) Öl(e) in den Zusammensetzungen in Konzentrationen von 0,05 bis 100 Gew.-% und stärker bevorzugt von 0,1 bis 40 Gew.-% und noch stärker bevorzugt von 0,1 bis 30 Gew.-%, noch besser von 0,5 bis 10 Gew.-%, bezogen auf das Gesamtgewicht der Zusammensetzung, vorliegt bzw. vorliegen.

8. Verfahren zum Schützen der Farbe künstlich gefärbter Keratinfasern, insbesondere menschlicher Keratinfasern und ganz besonders der Haare, gegenüber Wäsche, **dadurch gekennzeichnet, dass** man bei dem Verfahren auf die Fasern ein oder mehrere die Trocknung beschleunigende Öle oder eine Zusammensetzung, die in einem kosmetisch annehmbaren Medium ein oder mehrere die Trocknung beschleunigende Öle umfasst, aufträgt; wobei die die Trocknung beschleunigenden Öle ausgewählt sind aus Leinöl, chinesischem Holzöl, auch als Tungöl bezeichnet, Oiticicaöl, Vernoniaöl, Schlafmohnöl, Granatapfelkernöl, Calendulaöl, Perillaöl und deren Gemischen.

9. Verfahren nach Anspruch 8, bei dem die Keratinfasern mittels Oxidationsfärbung gefärbt werden.

10. Verfahren zur Färbung menschlicher Keratinfasern und ganz besonders der Haare, bei dem man auf die Fasern eine Zusammensetzung (A) zur Direktfärbung oder eine Zusammensetzung (A) zur Oxidation, gegebenenfalls in Gegenwart von einem oder mehreren Oxidationsmitteln, für eine Zeit aufträgt, die ausreicht, um die Farbe zu entwickeln, und dass man vor oder nach diesem Auftragen eine Zusammensetzung (B) aufträgt, die in einem kosmetisch annehmbaren Medium ein oder mehrere aus Leinöl, chinesischem Holzöl, auch als Tungöl bezeichnet, Oiticicaöl, Vernoniaöl, Schlafmohnöl, Granatapfelkernöl, Calendulaöl, Perillaöl und deren Gemischen ausgewählte die Trocknung beschleunigende Öle umfasst.

11. Verfahren nach Anspruch 10, wobei auf das Auftragen der Zusammensetzung (A) eine Spülung und/oder Trocknung der Keratinfasern folgt.

12. Verfahren nach einem der Ansprüche 10 oder 11, wobei auf das Auftragen der Zusammensetzung (B) eine Spülung und/oder Trocknung der Keratinfasern und/oder ein Erwärmen der Keratinfasern folgt.

13. Verfahren nach einem der Ansprüche 10 bis 12, wobei die Zusammensetzung (B), entweder direkt oder verzögert, nach dem Auftragen der Zusammensetzung (A) zur Direktfärbung oder zur Oxidationsfärbung aufgetragen wird und die Auftragungen der Zusammensetzung (B) zwischen zwei Färbungen wiederholt werden können.

14. Verfahren nach einem der Ansprüche 10 bis 13, wobei sich die Färbezusammensetzung (A) durch eine Mischung, unmittelbar im Moment der Verwendung, einer Färbezusammensetzung ($A_1$), die ein oder mehrere Direktfärbemittel umfasst, und einer Zusammensetzung ($A_2$), die ein oder mehrere Oxidationsmittel umfasst, ergibt.

15. Verfahren nach einem der Ansprüche 10 bis 14, wobei sich die Färbezusammensetzung (A) durch eine Mischung, unmittelbar im Moment der Verwendung, einer Färbezusammensetzung ($A_3$), die ein oder mehrere Oxidationsbasen und gegebenenfalls ein oder mehrere Kuppler und/oder ein oder mehrere Direktfärbemittel umfasst, und einer Zusammensetzung ($A_4$), die ein oder mehrere Oxidationsmittel umfasst, ergibt.

16. Verfahren zur Färbung von Keratinfasern, insbesondere menschlicher Keratinfasern und ganz besonders der Haare, bei dem man auf die Fasern eine Zusammensetzung (A) zur Direktfärbung für eine Zeit aufträgt, die ausreicht, um die Farbe zu entwickeln, wobei die Zusammensetzung (A) in einem kosmetisch annehmbaren Medium ein oder mehrere aus Leinöl ausgewählte die Trocknung beschleunigende Öle umfasst.

17. Kit, enthaltend mindestens einen ersten Bestandteil (oder mindestens ein erstes Kompartiment), umfassend eine Färbezusammensetzung (A), die in einem kosmetisch annehmbaren Medium ein oder mehrere aus Leinöl ausgewählte die Trocknung beschleunigende Öle und mindestens ein Direktfärbemittel umfasst, die mit einem zweiten Bestandteil (oder Kompartiment) vereinigt werden, das ein oder mehrere Oxidationsmittel umfasst.

## Claims

1. Use of one or more drying oils as an agent for wash-protecting the colour of artificially dyed keratin fibres, especially human keratin fibres, and more particularly the hair, in which the drying oils are chosen from linseed oil, China (or Canton) wood oil, also known as tung oil, oiticica oil, vernonia oil, poppyseed oil, pomegranate oil, calendula oil, perilla oil, and mixtures thereof.

2. Use according to Claim 1, **characterized in that** the keratin fibres are dyed by oxidation dyeing, in the presence of one or more oxidizing agents.

3. Use according to either of the preceding claims, **characterized in that** the drying oil is linseed oil.

4. Use according to one of the preceding claims, **characterized in that** the drying oil(s) is (are) a refined oil or refined oils.

5. Use according to one of the preceding claims, **characterized in that** the drying oil(s) is (are) partially polymerized by hot polymerization or blowing.

6. Use according to Claim 5, **characterized in that** the drying oil(s) is (are) chosen from linseed stand oils.

7. Use according to any one of the preceding claims, in which the drying oil(s) is (are) present in the compositions in concentrations ranging from 0.05% to 100% by weight, and more preferably from 0.1% to 40% by weight, and even more preferably from 0.1% to 30%, better still from 0.5% to 10% by weight, relative to the total weight of the composition.

8. Process for wash-protecting the colour of artificially dyed keratin fibres, especially human keratin fibres and more particularly the hair, **characterized in that** it consists in applying, to said fibres, one or more drying oils or a composition comprising, in a cosmetically acceptable medium, one or more drying oils; said drying oils being chosen from linseed oil, China (or Canton) wood oil, also known as tung oil, oiticica oil, vernonia oil, poppyseed oil, pomegranate oil, calendula oil, perilla oil, and mixtures thereof.

9. Process according to Claim 8, according to which the keratin fibres are dyed by oxidation dyeing.

10. Process for dyeing human keratin fibres and more particularly the hair, which consists in applying, to the fibres, a direct dye composition (A) or else an oxidation composition (A), optionally in the presence of one or more oxidizing agents, for a time sufficient to develop the colour, and preceding or following this application with the application of a composition (B) containing, in a cosmetically acceptable medium, one or more drying oils chosen from linseed oil, China (or Canton) wood oil, also known as tung oil, oiticica oil, vernonia oil, poppyseed oil, pomegranate oil, calendula oil, perilla oil, and mixtures thereof.

11. Process according to Claim 10, in which the application of the composition (A) is followed by rinsing and/or drying of the keratin fibres.

12. Process according to either of Claims 10 and 11, in which the application of the composition (B) is followed by rinsing and/or drying of the keratin fibres and/or heating of the keratin fibres.

13. Process according to any one of Claims 10 to 12, in which the composition (B) is applied after application of the direct or oxidation dye composition (A), either immediately, or after a delay, and it being possible for the applications of said composition (B) to be repeated between two dyeing operations.

14. Process according to any one of Claims 10 to 13, in which the dye composition (A) results from the mixing, at the time of use, of a dye composition ($A_1$) comprising one or more direct dyes and of a composition ($A_2$) comprising one or more oxidizing agents.

15. Process according to any one of Claims 10 to 14, in which the dye composition (A) results from the mixing, at the time of use, of a dye composition ($A_3$) comprising one or more oxidation bases and optionally one or more couplers and/or one or more direct dyes and of a composition ($A_4$) comprising one or more oxidizing agents.

16. Process for dyeing keratin fibres, in particular human keratin fibres and more particularly the hair, which consists in applying, to the fibres, a direct dye composition (A) for a time sufficient to develop the colour, said composition (A) comprising, in a cosmetically acceptable medium, one or more drying oils chosen from linseed oil.

17. Kit comprising at least one first component (or compartment) comprising a dye composition (A) comprising, in a cosmetically acceptable medium, one or more drying oils chosen from linseed oil; and at least one direct dye, combined with a second component (or compartment) comprising one or more oxidizing agents.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1312346 A
- EP 1240831 A
- EP 0337354 A
- FR 2270846 A
- FR 2383660
- FR 2598611
- FR 2470596
- FR 2519863
- FR 2505348
- FR 2542997
- EP 080976 A
- FR 2077143
- FR 2393573
- FR 2162025
- FR 2280361
- FR 2252840
- FR 2368508
- FR 1583363
- US 3227615 A
- US 2961347 A
- FR 2080759
- FR 2190406
- FR 2320330
- FR 2270846
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- EP 122324 A
- FR 1492597
- US 4131576 A
- US 3589578 A
- US 4031307 A
- FR 8516334 A
- US 4957732 A
- EP 186507 A
- EP 342834 A
- EP 412704 A
- EP 412707 A
- EP 640105 A
- WO 9500578 A
- EP 582152 A
- WO 9323009 A
- US 4693935 A
- US 4728571 A
- US 4972037 A
- DE 4424530
- DE 4424533
- DE 4402929
- DE 4420736
- WO 9523807 A
- WO 9407844 A
- EP 0646572 A
- WO 9516665 A
- FR 2673179
- EP 0227994 A
- WO 9424097 A
- WO 9410131 A
- US 4874554 A
- US 4137180 A
- EP 486135 A
- WO 9311103 A
- US 4103145 A
- US 4308878 A
- US 5983903 A
- US 5957140 A
- US 5494058 A
- FR 2692572
- WO 9501772 A
- WO 9515144 A
- EP 714954 A
- GB 1026978 A
- GB 1153196 A
- DE 2359399
- JP 63169571 A
- JP 05163124 B
- EP 0770375 A
- WO 9615765 A
- FR 2750048 A
- DE 3843892
- DE 4133957
- WO 9408969 A

- WO 9408970 A
- FR 2733749 A
- DE 19543988
- FR 2886136

**Littérature non-brevet citée dans la description**


- **P.D. DORGAN.** *Drug and Cosmetic Industry,* Décembre 1983, 30-33
- **WALTER NOLL.** Chemistry and Technology of Silicones. Academie Press, 1968
- **TODD ; BYERS.** Volatile Silicone fluids for cosmetics. *Cosmetics and toiletries,* vol. 91 (76), 27-32